# EUROPÄISCHE PATENTANMELDUNG

(11) **EP 2 626 410 A1**
(43) Veröffentlichungstag der Anmeldung: **14.08.2013**
(21) Anmeldenummer: 12000816.4
(22) Anmeldetag: 08.02.2012
(51) Int. Cl.: C12M 1/12

(54) **Vorrichtung und Verfahren zur Expansion und Ernte von adhärenten Zellinien**

(71) Anmelder: Technische Hochshule Mittelhessen, 35390 Giessen (DE)
(72) Erfinder: Justice, Christiane, 35614 Asslar (DE); Ebrahimi, Mehrdad, 35394 Giessen (DE); Pino-Grace, Pablo, 35392 Giessen (DE); Czermak, Peter, 35576 Wetzlar (DE)
(74) Vertreter: Stumpf, Peter

(57) **Zusammenfassung**

Die vorliegende Erfindung betrifft eine Vorrichtung integriert in einem rührbaren Bioreaktor und ein Verfahren zur Expansion und Ernte von adhärent wachsenden Zellen oder von Produkten, die aus Kulturen adhärent wachsender Zellen hergestellt werden, unter Einbindung von Process Analytical Technology (PAT)-Elementen.

## Beschreibung

Die vorliegende Erfindung betrifft ein integriertes vereinfachtes System zur Expansion und Ernte von adhärent wachsenden Zelllinien, wie z.B. humane mesenchymale Stammzellen für die Herstellung zelltherapeutischer Implantate in einem System. Auch für biopharmazeutische Produktionsprozesse, wie z.B. Virenproduktion durch Vero-Zellen sind denkbar sowie Antikörper und andere Proteine durch adhärent wachsende Zelllinien. Im Folgenden werden Produkte für den Einsatz in der Zelltherapie zu "Zellen" zusammengefasst und für Produkte der biopharmazeutischen Technologie zu "Proteine".

In der Medizin ist der Bedarf biopharmazeutischer Produkte sowie an Zellen für den Einsatz in der Zelltherapie gestiegen. Es besteht ein hoher Bedarf an Produkten, deren Herstellungsprozesse den GMP-Richtlinien gerecht werden müssen und die Anforderungen der Behörden (FDA, EMEA) erfüllen müssen.

Entscheidend für die Produktionsprozesse ist eine hohe Produktqualität. Im Falle der Produktion von Zellen für den Einsatz in der Zelltherapie müssen die Zellen hohe Qualitätsansprüche erfüllen, wie hohe Vitalität, stabiles Expressionsverhalten und Differenzierbarkeit. Analog ist bei Biopharmazeutika entsprechen die hohe Qualität des Proteins erforderlich. Auch ist die Prozesseffizienz von großer Bedeutung. Einfache Systeme mit möglichst geringer Komplexität, wenig Peripherie und Anzahl an Bauteilen sind wünschenswert um Kontaminationsrisiken zu reduzieren sowie die Prozessvalidierung zu vereinfachen. Der Einsatz von Einwegartikeln ist besonders erstrebenswert, um die Prozesskosten zu reduzieren und den Zulassungsprozess zu vereinfachen. Durch die PAT-Initiative der FDA in 2004 ist eine Prozessüberwachung mittels entsprechender on-line und off-line- Sensorik erforderlich. Bisher sind hierfür für den Expansionsprozess Methoden zur Bestimmung von Metaboliten, Nährstoffen sowie Zelldichten möglich. Für die Produktion von Zellen ist zusätzlich die Überwachung des Ablöseprozesses der Zellen wünschenswert. Eine Überwachung von z.B. Virusfreigabe durch Platzen von Zellen durch die Anwendung der Impedanzspektroskopie ist bereits bekannt.

Produktionsprozesse werden in zwei große Abschnitte unterteilt: Up-Stream und Downs-Stream. Im Up-Stream werden die Zellen ausgehend von einem Inokulum expandiert, während im Downstream die eigentliche Produktgewinnung durch Abtrennung und Aufreinigung erfolgt. Für diese Erfindung sind der Up-Stream sowie die Produktabtrennung von Bedeutung, die Aufreinigung wird nicht betrachtet.

Im Up-Stream erfolgt die Massenkultivierung für die Produktion von Zellen bzw. Biopharmazeutika zumeist mit stringent adhärent wachsenden Zellen und stellt eine Herausforderung dar. Adhärent wachsende Zellen benötigen eine Oberfläche für ihr Wachstum und sind sehr empfindlich gegenüber Scherbelastungen und Änderungen der Wachstumsumgebung. Erforderlich sind daher scherarme Prozesse mit der Möglichkeit das Medium bei Bedarf einfach tauschen zu können.

Im Stand der Technik werden im Up-Stream statische Systeme mit zweidimensionaler Oberfläche, wie Well-Platten, T-Flaschen und gasdurchlässige Beutel verwendet. Nachteilig an statischen Bioreaktoren, bei denen der Transport von Nährstoffe, Zellen, Metaboliten, Sauerstoff und anderen Stoffen nur per Diffusion erfolgt, ist, dass sie nur für geringe Zelldichten und niedrige Ausbeuten an Produkt geeignet sind. Zudem sind diese Systeme inhomogen.

Auch dynamische Systeme wie Trägerbasierte gerührte Systeme als Batch und Perfusionssysteme für die Produktion von Biopharmazeutika sind bekannt. Die Oberfläche wird hier in Form von Carrier bereitgestellt.

Für alle Systeme gilt jedoch, dass das Produkt (Zellen/ Protein) von der Trägersubstanz (Oberfläche, Carrier) getrennt werden muss. Weiterhin ist ein regelmäßiger Austausch des Mediums erforderlich, um die Produktqualität sichern zu können. Bei gerührten Perfusionssystemen, die diesen Mediumwechsel ermöglichen, ist bisher jedoch eine Vorrichtung außerhalb des Reaktors für die Abtrennung der Carrier von der Flüssigkeit erforderlich. Hier treten jedoch häufig Probleme mit Verblockung der Abtrennsysteme auf und durch die Anbringung der Abtrennvorrichtung ist die Kontaminationsgefahr erhöht und Sterilisationsprozesse erschwert. Zusätzlich müssen die Zellen weitere Elemente durchströmen und sind dort möglicherweise hohen Scherkräften ausgesetzt. Alternativ können die Carrier vor einem Mediumwechsel sedimentiert werden lassen, was jedoch häufig zur Bildung von Aggregaten und Problemen mit der Sensorik führt. Weiterhin wird so der Prozess unterbrochen und kann nur schwer automatisiert werden. Eine online-Überwachung des Zellablösevorgangs ist bisher nicht bekannt, jedoch wünschenswert.

Auch sind Festbettsysteme bekannt, die eine scherarme Kultivierung der Zellen ermöglichen und einen Perfusionsbetrieb ohne zusätzliche Periphere erlauben, jedoch ist die Homogenität im System nicht sicher gewährleistet und eine Überwachung der Prozesse nur schwer möglich.

### Aufgabe

Aufgabe der Erfindung ist es, die Nachteile im Stand er Technik zu beseitigen und ein Verfahren und eine Vorrichtung zur Durchführung des Verfahrens bereitzustellen, das zur einfachen Herstellung und Abtrennung von Zellen/ Protein geeignet ist und bei dem möglichst wenig Systemkomponenten erforderlich sind. Auch soll die Prozessüberwachung nach PAT verbessert werden.

### Lösung

Die Aufgabe wird gemäß den Ansprüchen gelöst, indem eine Vorrichtung und ein Verfahren zur Expansion und Ernte von Zellen bzw. Protein bereitgestellt werden, das eine einfache integrierte Lösung im Rührreaktor selbst erlaubt. So kann das Produkt (Zelle/ Protein) einfach und mit hoher Qualität hergestellt und im gleichen System ohne weitere Peripherie von den Trägern getrennt werden. Es entstehen keine Probleme mit Verblockung der Abtrennvorrichtung. Durch Integration der Impedanzspektroskopie kann der Ablöseprozess der Zellen von den Trägern bei der Zellernte überwacht und somit optimiert werden.

Weiterhin ist durch die integrierte Trennung eine scherarme blasenfreie Begasung der Zellen durch externe Membranmodule möglich. Sollte Blasenbegasung jedoch erwünscht sein, so kann ein Begasungselement oberhalb des Siebes integriert werden. Ebenso kann eine optimale Versorgung der Zellen mit Medium durch Perfusion ermöglicht werden. Dies dient zur besseren Zellversorgung ebenso wie im Fall der biopharmazeutischen Prozesse zur kontinuierlichen Abtrennung des Proteins und damit Reduktion von Produktinhibierung. Auch kann dadurch das Produkt schneller gekühlt und somit stabilisiert werden, da eventuell auftretende Proteasen ihre Wirkung nicht entfalten können.

Durch die Verwendung eines Ablasses am Reaktorboden kann der Einsatz einer Pumpe durch die Gravitation eliminiert werden. So werden die Zellen weniger Scherbelastung ausgesetzt. Eine Vakuumpumpe an Abfall bzw. Erntegefäß kann bei zu geringen Druckunterschieden Abhilfe schaffen.

Da während des Perfusionsprozesses und auch bei der Ernte die Abtrennvorrichtung (Sieb) kontinuierlich angeströmt wird, wird eine Kuchenbildung und Verblockung des Siebes vermieden.

Das integrierte Abtrennsystem besteht aus einem planen oder gewölbtem Sieb mit geringer Maschenweite (z.B. ca. 50-500µm), welche so gewählt ist, dass die Zellen es passieren können, Trägersysteme wie Microcarrier jedoch nicht. Das Sieb, sowie die evtl. notwendige Fassung und Dichtung bestehen aus sterilisierbarem, bioinertem Material, dass von der FDA zugelassen ist. Die Einheit wird unterhalb des Rührelementes, jedoch oberhalb des Spargers integriert. Dies erfolgt durch eine Spannvorrichtung, die über eine Dichtung fixiert wird oder aber das Sieb wird bei Einwegsystemen bei der Produktion während der Herstellung fest installiert/verbunden. Beispielsweise kann eine Wellenführung auf dem Sieb aufgesetzt werden, die für eine stabilere Wellenführung erforderlich sein könnte. Das Sieb wird durch den Rührer angeströmt, was Verblockungsprobleme eliminiert. Unterhalb des Siebes befindet sich ein Auslass, durch den Medium, geerntete Zellsuspension und Produkt entnommen werden können. Auch wäre es beispielsweise möglich, eine rotierende Dichtung in den das Sieb zu integrieren, sodass eine Rührerwelle dieses durchstößt und so auch unterhalb des Siebes ein Rührer eingebracht werden könnte. Somit könnte eine bessere Vermischung der Flüssigkeit auch unterhalb des Siebes erreicht werden. Die Verwendung verschiedenere Rührertypen würde zu unterschiedlichen Strömungsprofilen führen und eine andere Zirkulation der Flüssigkeit verursachen. Der Rührertyp kann je nach Prozessziel variiert werden. Gängige Beispiele für Rührer für die Kultivierung von Zellen sind Marine-Impeller, Propeller und Scheibenrührer.

Durch die Messung von Nährstoffen, Medium, Metaboliten, Sauerstoff und anderen Stoffen im Bioreaktor kann deren Transport in den Bioreaktor kontrolliert ein Austausch ermöglicht werden. Dieses Verfahren wird als Batch-Verfahren oder kontinuierlich durchgeführt, wobei höhere Zelldichten, im Vergleich zu statischen Methoden erreicht werden. Das Scale-up dieser Prozesse ist auf Grund des verbesserten und automatisierten Stofftransportesrelativ einfach.

Die Herstellung von Zellen und Protein erfolgt erfindungsgemäß in einem gerührten Reaktor oder gerührten Einwegreaktor mit Bodenauslass und integrierter Abtrennvorrichtung. Die Prozesskontrolle erfolgt vorzugsweise online unter Nutzung der Impedanzspektroskopie. Dies ermöglicht es, die Zellzahl der Zelllinie, deren Vitalität und eventuell Produktionsaktivität zu kontrollieren und so den gesamten Kultivierungsverlauf zu automatisieren, dass ein exakter Erntezeitpunkt/Abbruchzeitpunkt festlegbar ist.

Erfindungsgemäß sind Einwegreaktoren und Einweg-Rührreaktoren Vorrichtungen, die für Produktionsprozesse von scherempfindlichen Zellen (Suspensions- und adhärente Kulturen) und pharmazeutischen Produkten, ebenso wie zur Herstellung von Medien, Inokulum und Mischprozessen eingesetzt werden.

Der Vorteil der Verwendung von Einwegsystemen liegt in der Elimination von *Sterilization in Place* (SIP), *Cleaning in Place* (CIP) und somit der Reduktion von Prozesszeiten. Auch wird das Risiko von Kreuzkontaminationen reduziert, der Qualifizierungs- und Validierungsaufwand eines Prozesses wird reduziert, was in der Gesamtbetrachtung Kostenersparnisse von bis zu 50% ermöglich. Die Einbindung des Siebes ermöglicht nach wie vor eine problemlose Durchführung von CIP, sowohl ober- als auch unterhalb des Siebes, sowie des Siebes selbst. Es ist keine CIP-Validierung für ein externes Abtrennsystem notwendig.

Die Vorrichtung zur Expansion und Ernte von Zellen bzw. Protein umfasst einen gerührten Bioreaktor(R1) mit integrierter Abtrennvorrichtung sowie Bodenauslass. Der Bioreaktor wird durch ein Prozessleitsystem (S1) gesteuert, um optimale Bedingungen für das Zellwachstum/ Produktion zu ermöglichen.

Es sind zwei Prozessmodi möglich, als Batch- und Perfusionsbetrieb. Auch ist eine Zufütterung konzentrierter Lösungen möglich, jedoch sollte das Reaktionsvolumen möglichst konstant gehalten werden, sodass die Carrierkonzentration sich nicht signifikant ändert, um nachteilige Effekte durch erhöhte Scherbelastung zu vermeiden.

Im Batch-Prozess ist der Reaktor (R1) an die Steuereinheit (S1) angeschlossen. Für die Ernte von Zellen (für die Zelltherapie) werden Gefäße mit Pufferlösung (G2), Enzymlösung (G1) und Medium (G3) sowie ein Abfall- (G5) und Erntegefäß (G4) über Schlauchsysteme an den Reaktor angeschlossen. Der Flüssigkeitstransport wird über Ventile (V1-V5) und Pumpen (P1-P2) gesteuert. Die Begasung des Systems mit Kohlendioxid zur pH-Korrektur oder mit Luft zur Versorgung mit Sauerstoff kann scherarm und blasenfrei über eine externe Membran (M1) erfolgen oder aber auch durch Begasung über ein im System integrierten Sparger unterhalb der Abtrennvorrichtung. Hier ist Begasung mit reinem Sauerstoff denkbar, da so nur wenig Blasenbildung erforderlich ist und der Sauerstoff sich direkt löst. Man erhält somit eine effiziente annähernd scherarme blasenfreie Begasung umsetzen.

Im Falle der Produktion von Proteinen sind Gefäße G1-2 nicht erforderlich, G4jedoch nach wie vor als Erntegefäß. Gefäße G1-4 können jedoch andere Lösungen enthalten, die beispielsweise für die Produktstabilität oder erhöhte Ausbeute hilfreich sind.

Im Fed-Batch-Prozess ist dann ein weiteres Vorratsgefäß G3 mit konditioniertem Medium an das System gekoppelt, aus dem über eine Pumpe (P1) und durch ein Ventil (V3) gesteuert die Fütterung erfolgt. Die Begasung erfolgt gleich wie beim Batch-Prozess

Im Perfusionsprozess kann kontinuierlich Medium abgenommen und frisches zugeführt werden. Es ermöglicht so für Proteinproduktionen extrazellulärer Produkte eine Abtrennung des Produktes vom Reaktionsvolumen, wodurch Produktinhibierungen vermieden werden können. Außerdem kann das Produkt umgehend gekühlt und somit stabilisiert werden. Der große Vorteil liegt hier in der einfachen Entnahme von Carrier- und zellfreier reaktorbrühe.

In allen Varianten wird der Prozess über Sensorik für pH, Sauerstoff, Impedanz und Temperatur überwacht und über das Prozessleitsystem gesteuert. Das Prozessleitsystem wird vorzugsweise mit einer Software, z.B.Labvision (Hightec Zang, Herzogenrath, D.) gesteuert.

Die Kultivierung der Zellen (als Produkt oder als Produktionszelle) erfolgt wie im Stand der Technik auf geeigneten Trägern geringer Dichte, um eine einfache Aufwirbelung der Carrier bei geringem Leistungseintrag zu ermöglichen. Diese können sowohl poröse als auch nicht-poröse Carrier mit geeigneter Oberfläche sein.

Das erfindungsgemäße System weist eine erhöhte Prozesssicherheit auf und ermöglicht so einen automatischen Mediumwechsel, ohne dass die Carrier sedimentiert werden lassen müssen. Auch ist die Zellernte bzw. Abtrennung von Proteinhaltiger Lösung automatisch möglich, ohne dass gesonderte Peripherie erforderlich ist. Der Prozess wird anhand von Zelldichte und Nährstoffkonzentrationen, wie z.B. Glukose, oder Proteinkonzentrationen überwacht und anhand der Daten der analytischen Instrumente der Erntezeitpunkt bestimmt. Erstmals ist eine optimale Kontrolle des gesamten Produktionsprozesses der Zellen (Animpfen, Expansion und Ernte von Zellen bzw. Protein) in einem System möglich, wobei jeder dieser Prozesse anhand von online-Analytik nach PAT überwacht wird. Dies ist für die Durchführung eines GMP-gerechten Prozesses zur Produktion von Biopharmazeutischen Produkten mit adhärenten Zellen ein großer Fortschritt.

### Aufbau des Bioreaktorsystems

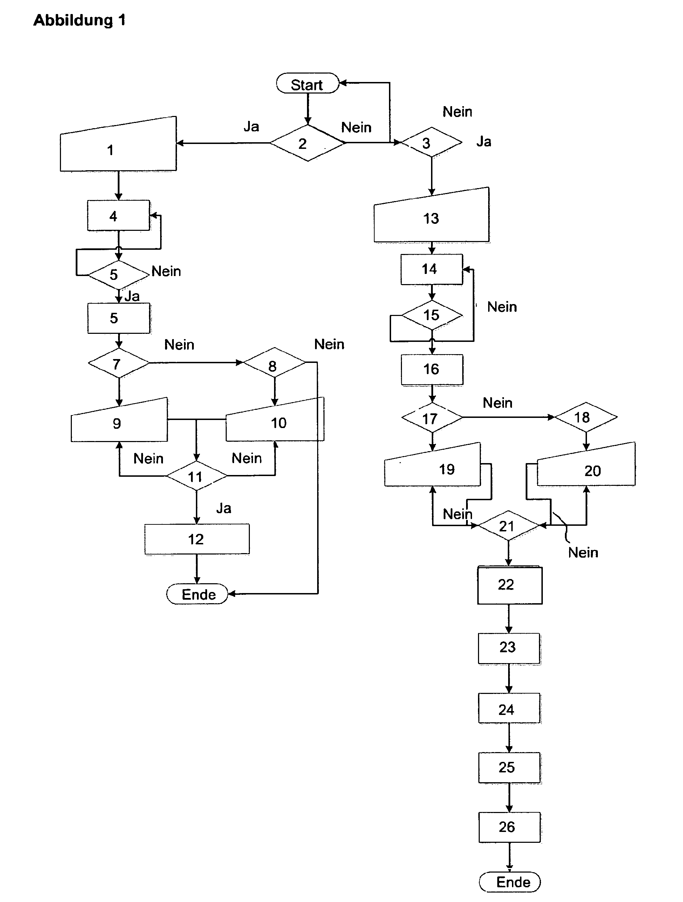

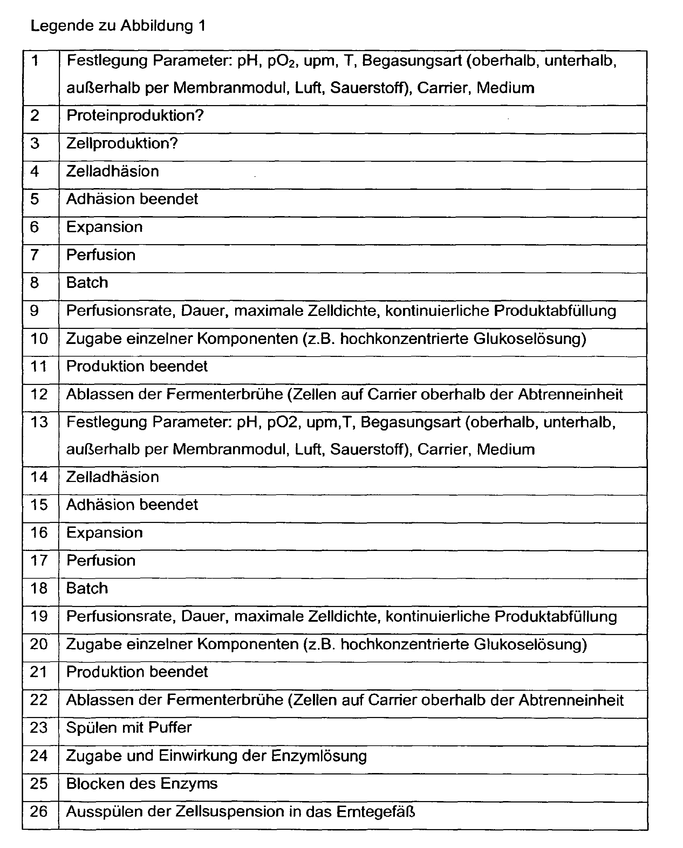

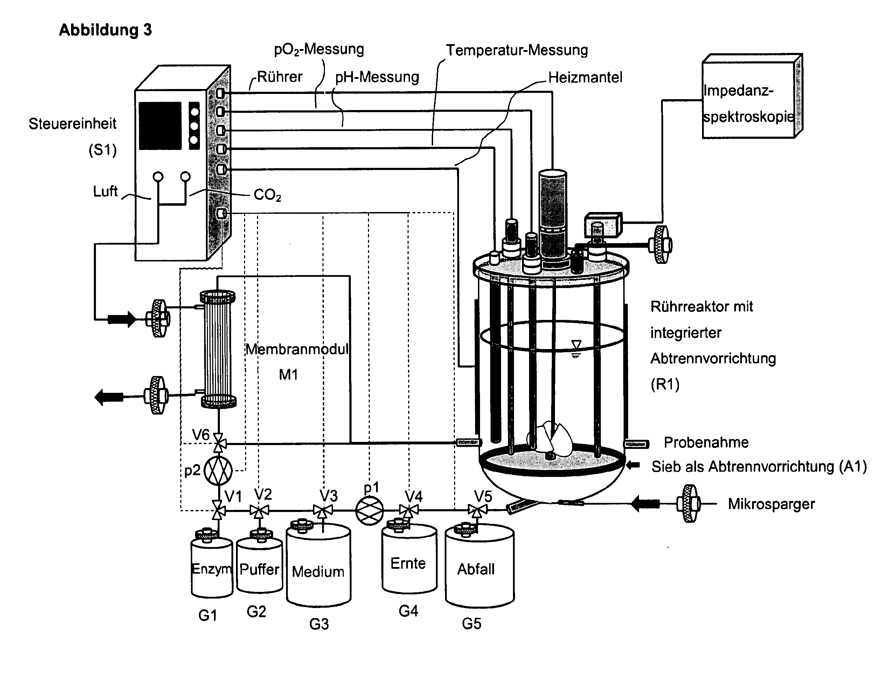

Der Prozessablauf ist schematisch in Abbildung 1 dargestellt. Folgend werden Teile dieses Ablaufs detaillierter beschrieben. Abbildung 3 zeigt die schematische Darstellung eines erfindungsgemäßen Ausführungsbeispiels.

### Prozesssteuerung

Für die Prozesssteuerung ist die Überwachung von Parametern wie O₂, CO₂, pH, Temperatur, Glukose-, Laktat-, sowie die Zellkonzentration entscheidend. Ausgenommen der Glukose- und Laktatkonzentration ist für die genannten Parameter entsprechende online-Analytik auf dem Markt verfügbar und etabliert. Die Impedanzspektroskopie wird für die Bestimmung von Zellkonzentration sowie Zellgröße verwendet. Ihre Daten ermöglichen Aussagen über Prozessänderungen (Leitfähigkeit, Zellgröße, ...)

### Animpfprozess

Das Animpfen mit einer Starterkultur aus Zellen erfolgt manuell direkt in den Reaktor (R1). Mit der Impedanzsonde wird die genaue Zelldicht beim Animpfen beobachtet und dokumentiert. Während des Animpfprozesses werden die Zellen bei sehr geringen Rührerumdrehungen zusammen mit den Carriern in Schwebe gehalten und können so adhärieren. Hierbei befindet sich unterhalb des Siebes ein Luftkissen, welches das Durchdringen der Zellen unterhalb des Siebes vermeidet. Erreicht das Signal der Impedanzspektroskopie ein konstantes Signal für die kritische Frequenz, so ist der Adhäsionsvorgang weitestgehend abgeschlossen.

### Expansionsprozess

Die Expansion der Stammzellen aus der Starterkultur wird online durch Messung der Glukosekonzentration anhand einer der Prozesssteuerung hinterlegten Minimalkonzentration überwacht und bei Bedarf ein Mediumwechsel automatisch eingeleitet und durchgeführt. Dies führt zu einer Vollautomatisierung des Systems und zu einer Erhöhung der Prozesssicherheit durch permanente online-Überwachung der Lebendzellmasse und der Nährstoffversorgung (Glukose). Alternativ wird der Mediumwechsel auch manuell eingeleitet.

Im Verlauf des Expansionsprozesses nimmt die Zellzahl exponentiell zu. Dieses Wachstum ist mittels der Impedanzsonde beobachtbar und die Signalinterpretation gut umsetzbar, da die Signale linear mit der Zellzahl korrelieren. Weiterhin wird offline oder online eine Glukosekonzentrationsbestimmung durchgeführt. Diese Information bestätigt dann die jeweils notwendige Perfusionsrate oder aber die Zugabemenge an konzentrierter Glukoselösung. Ist die gewünschte Zellzahl erreicht, so wird über die Prozesssteuerung der Ernteprozess eingeleitet.

### Ernteprozess

Ist der Ernteprozess nach der Expansion in z.B. dem Rührreaktor mit Sieb eingeleitet, so ändert sich das Signal während des Einwirkens des Enzyms, da sich die Zellgröße beim Ablösen der Zellen von dem Trägermaterial ändert, ebenso wie sich die Membraneigenschaften ändern. Werden die Zellen ausgeströmt, so sinkt das Signal während des Prozesses, bis ein relativ konstanter Wert erreicht wird.

### Beispiele

### Beispiel 1: Experimentelle Daten

Untersuchungen werden in einem Einwegbioreaktor mit Bodenauslass mit einem Volumen von 31 durchgeführt. Integriert wird ein Sieb aus Edelstahl mit einer Maschenweite von 100µm, welches in eine Polycarbonatfassung eingespannt wird und durch einen Dichtungsring im Reaktor am Übergang zwischen Klöpperboden und zylindrischem Teil des Reaktors festgesetzt wird.

Die verwendete Zelllinie ist eine adhärent wachsende, genetisch veränderte, sehr empfindliche humane mesenchymale Stammzelllinie (hMSC-TERT). Die Wachstumsoberfläche wird durch die Verwendung von RapidCell-Carriern mit Glasoberfläche bereitgestellt (MP Biomedicals, Dichte 1,03 g/ml, Größe 150-210 µm und spezifischer Oberfläche von 325 cm²/g).

Das verwendete Membranmodul ist ein in der Dialyse gängig verwendetes Modul. Die Versuche werden unter unsterilen Bedingungen durchgeführt.

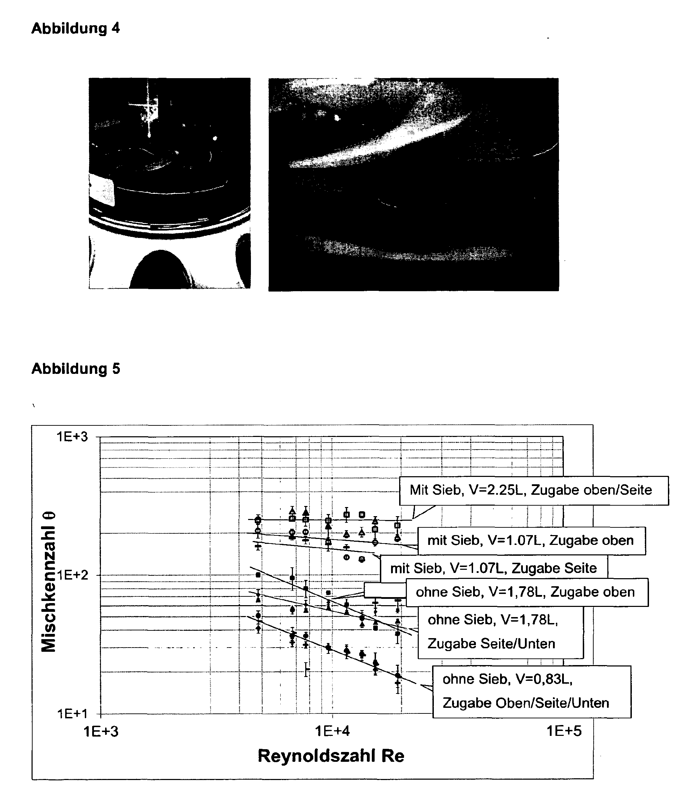

Abb. 4 zeigt das System in Ausschnitten anhand von Fotoaufnahmen des in den Reaktor eingebrachten Siebs unterhalb des Rührers.

### Beispiel 2: Homogenität

Für die Kultivierung von adhärenten Systemen ist eine homogene Umgebung für die Nährstoffversorgung und gleichmäßige Versorgung der Zellen erforderlich. Die Homogenität des Systems wird anhand von Mischzeitversuchen mit Farbumschlag untersucht. Als Mischgüte wird 95% festgelegt (für das gesamte System: ober- und unterhalb des Systems). Es werden verschiedene Zugabeorte der Entfärbelösung untersucht. Bei der Zugabe unterhalb des Siebes erfolgt annähend keine Durchmischung.

Abb. 5 zeigt Mischkennzahlen des Reaktorsystems mit und ohne eingebrachtes Sieb. Es ist deutlich zu erkennen, dass die Einbringung des Siebes zur Erhöhung der Mischkennzahl führt, bedingt durch die schlechte Durchmischung unterhalb des Siebes. Das eigentliche Reaktionsvolumen oberhalb des Siebes wird jedoch sehr gut durchmisch. Die Zugabe der Entfärbelösung von der Seite zeigt sich als vorteilhaft. Entsprechend erfolgt auch im beschriebenen System die Zugabe von Flüssigkeiten über einen seitlichen Port. Die Daten geben eindeutige Information darüber, dass im Reaktorsystem auch bei geringen Leistungseinträgen homogene Bedingungen vorliegen.

### Beispiel 3: Verweilzeituntersuchungen

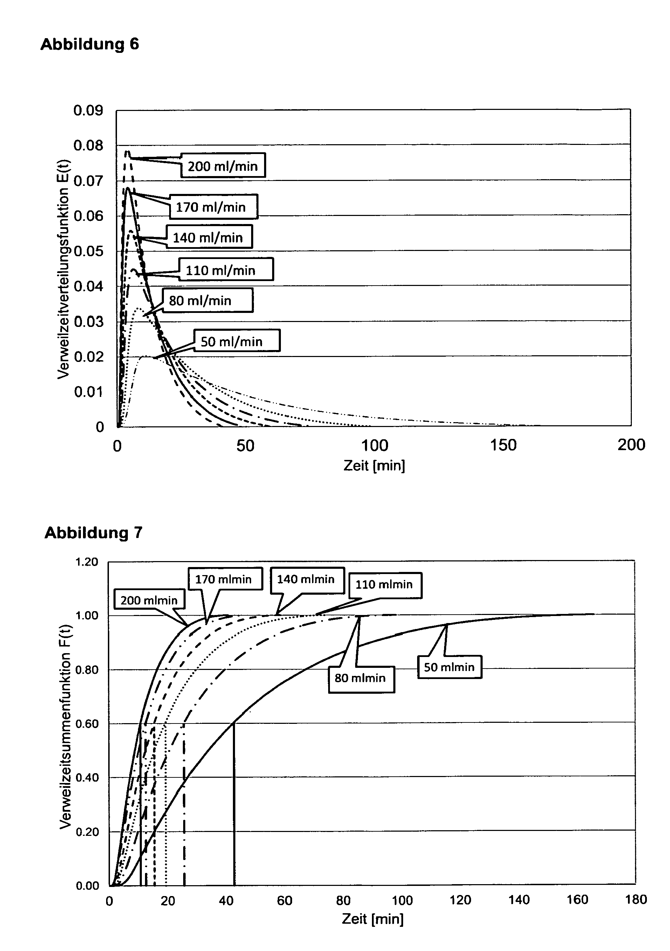

Wie für das System beschrieben, sind Perfusionsprozesse hilfreich, um ggf. notwendige Medienwechsel zu vereinfachen oder eine kontinuierliche Produktabtrennung zu ermöglichen. Für die Untersuchungen wird als Tracer eine Salzlösung hoher Leitfähigkeit verwendet. Die Rührergeschwindigkeit zeigt keinen signifikanten Einfluss auf die Verweilzeiten. Die Abbildungen 6 und 7 zeigen die Verweilzeitdichte- bzw. Verweilzeitsummenfunktion des Systems mit integriertem Sieb, in denen jeweils auch die mittleren Verweilzeiten gekennzeichnet sind. Deutlich zu sehen ist die Steigerung der Verweilzeit mit geringerem Volumenstrom. Diese Verweilzeiten dienen als Basis für die Berechnungen der erforderlichen Perfusionsraten in Abhängigkeit der gewählten Zelllinie und der Prozessparameter. Die Verweilzeit erhöht sich im Vergleich zum System ohne integriertes Sieb jedoch nur geringfügig, hier sind minimale Effekte der fehlenden Durchmischung durch den Rührer unterhalb des Siebs verantwortlich.

### Beispiel 4: Sauerstoffversorgung über ein Membranmodul

Untersuchungen zur Sauerstofftransferrate mit dem Modul dienen der Prüfung der ausreichenden Sauerstoffversorgung. Bei einer Umdrehungsgeschwindigkeit des Rührers von 100 upm werden bei verschiedenen Volumenströmen des Mediums kla-Bestimmungen durchgeführt. Die Strömungsgeschwindigkeit des Gases hat im Gegensatz zum Druck keinen Einfluss. Der Druck wird hier bei 0,5 bar festgelegt. Die Daten sind die Sauerstofftransferraten über das Modul (Oberflächenbegasung wurde ermittelt und jeweils subtrahiert).

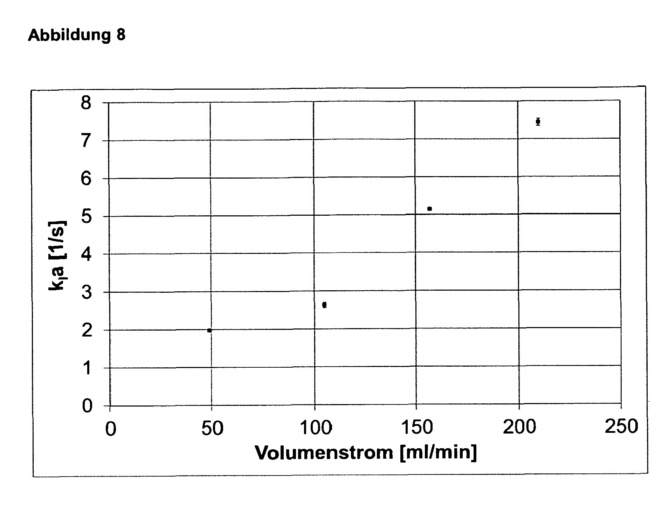

Abb. 8 zeigt die Sauerstofftransferrate im System mit integriertem Sieb bei 100 upm und 0,5bar Druck im Modul. Die ermittelten Sauerstofftransferraten sind größer 2 s⁻¹ und zeigen, dass eine Sauerstoffversorgung von tierischen Zellen problemlos möglich ist.

### Beispiel 5: Animpfprozess

Es kann gezeigt werden, dass auch durch kontinuierliches Rühren, die Zelladhäsion gleichmäßig verteilt auf den Carriern sehr gut umsetzbar ist. Es bildet sich unterhalb des Siebs ein Luftkissen, wodurch Medium und Zellen oberhalb des Siebes bleiben. Es werden mikroskopische Aufnahmen getätigt, um den Animpfverlauf optisch beurteilen zu können.

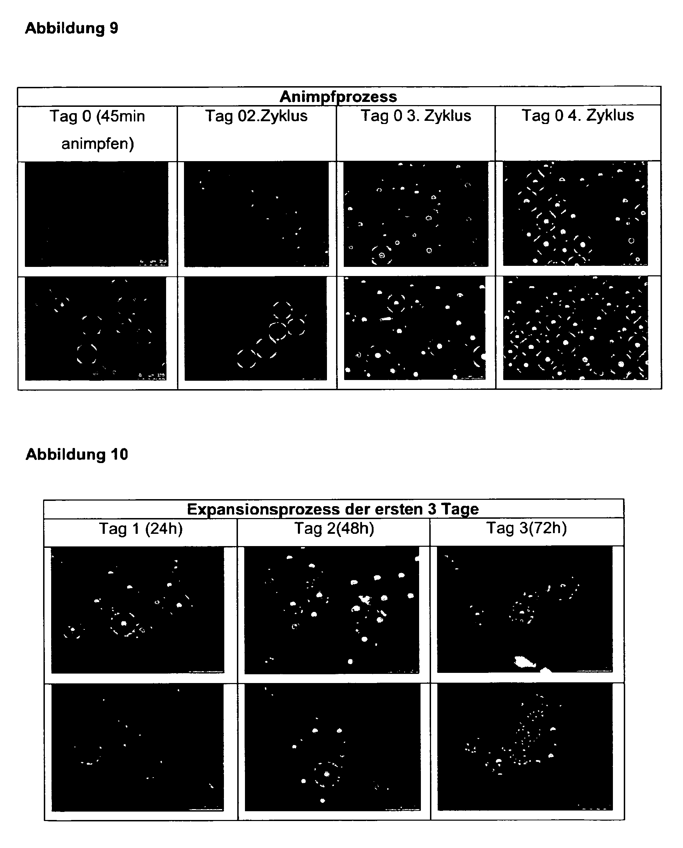

Abb. 9 zeigt den Verlauf des Animpfprozesses im System mit integriertem Sieb. Die Zellkerne werden mittels eines DNA-interkalierenden Fluoreszenzfarbstoffes grün gefärbt.

Weiterhin kann in den ersten Tagen der Expansionsphase der Prozess verfolgt werden. Abb. 10 zeigt mikroskopische Aufnahmen des Expansionsprozesses während der ersten drei Tage der Kultivierung.

### Beispiel 6: Ernteprozess

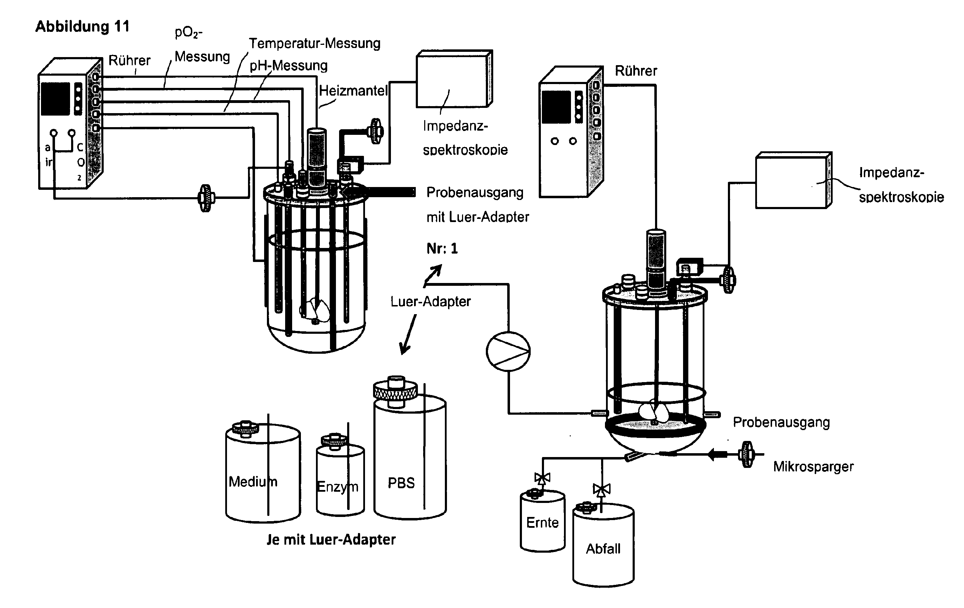

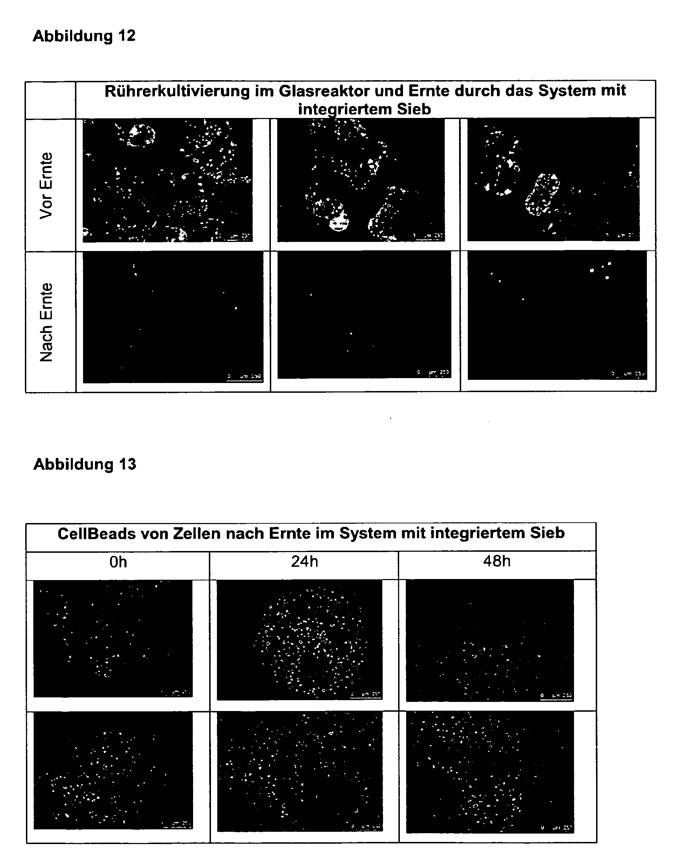

Da bisher nur unsterile Systeme zur Verfügung stehen, wird ein Glasreaktor annähernd gleicher Bauart für die Expansion der Zellen verwendet und die sehr konfluent bewachsenen Carrier (Worst-Case-Betrachtung übervoller Carrier) in das System mit einer Pumpe überführt. Abb. 11 zeigt eine schematische Darstellung des Prozessablaufs zur Ernte im System mit integriertem Sieb. Hier werden die Zellen nach dem beschriebenen Schema im System mit integrierter Abtrennvorrichtung geerntet. Abb. 12 zeigt die Carrier vor und nach der Ernte (ebenfalls die Zellkerne durch Fluoreszenzfarbstoff grün sichtbar gemacht).

Die sehr bewachsenen und auch z.T. sehr aggregierten und verwachsenen Carrier können gut von den Zellen abgetrennt werden. In der Zellsuspension sind auch nach Zentrifugation keine Carrier erkennbar. Die Zellablöse ist erfolgreich und auch die Zellqualität ist zufriedenstellend.

Die Qualitätskontrolle der Zellen umfasst die Beurteilung der Vitalität der Zellen vor und nach einem Verkapselungsprozess. Abb. 13 zeigt dazu mikroskopische Aufnahmen der CellBeads von Zellen, die im System mit Sieb geerntet wurden.

Es kann gezeigt werden, dass die CellBeads (verkapselte Zellen) hoch vital sind (>90%). Geerntete Zellen von statischen Kulturen zeigen vergleichbare Ergebnisse. Abb. 13 zeigt solche CellBeads von geernteten Zellen aus dem beschriebenen System im integrierten Sieb. Diese Verkapselung dient beim Einsatz der Zellen in der Zelltherapie dem Schutz vor Immunreaktionen des Patienten. Der Herstellungsprozess ist jedoch für die Zellen ein großer Stressfaktor und die Vitalität der Zellen ein wichtiges Merkmal für die Zellqualität. Die Zellernte ist einfach, schnell, effizient und für das gesamte Reaktionsvolumen problemlos möglich.

### Beschreibung der Abbildungen

### Abkürzungsliste der Abbildungen:

(R1) Rührreaktor mit integrierter Abtrennvorrichtung
(P1) Pumpe
(P2) Pumpe
(M1) Membranmodul
(S1) Steuereinheit
(V1) Ventil
(V2) Ventil
(V3) Ventil
(V4) Ventil
(V5) Ventil
(V6) Ventil
(W1) Waage
(G1) Gefäß
(G2) Gefäß
(G3) Gefäß
(G4) Gefäß
(G5) Gefäß

**Abbildung 1** zeigt eine schematische Darstellung des Prozessablaufs.

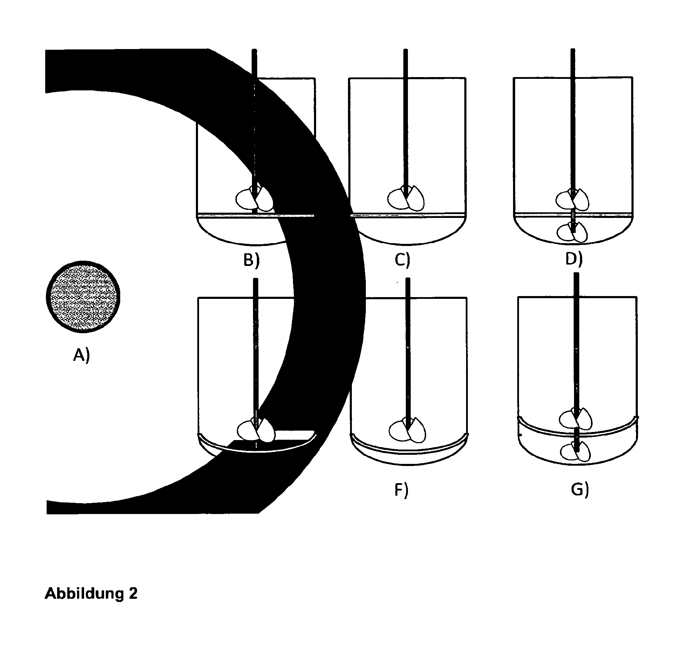

**Abbildung 2** zeigt eine schematische Darstellung der erfindungsgemäßen Einheit (Abtrennvorrichtung), welche in das Reaktorsystem eingebracht wird. Aufsicht: Sieb aus geeignetem Material in einer Fassung, welche durch eine Dichtung im System eingespannt wird oder aber das Sieb unmittelbar im Reaktorgefäß selbst mit eingebunden ist (z.B. bei Einwegreaktoren im Spritzguss integriert wird). Die Maschenweite des Siebes ist abhängig vom gewählten Carriersystem und der Zelllinie. A) zeigt das Sieb in der Aufsicht, B) in planer Form eingebracht in den Reaktor mit Führung für die Rührerwelle, C) wie B nur ohne Führung für die Rührerwelle, D) als Beispiel mit zwei Rührern, je einer ober- und unterhalb des Siebes mit Gleitringdichtung für die Welle durch das Sieb, E) ist das Sieb in nichtplaner gewölbter Form mit Führung für die Welle, F) wie E aber ohne Wellenführung, G) wie D aber für gewölbte Siebform.

**Abbildung 3** zeigt eine schematische Darstellung des erfindungsgemäßen Verfahrens.

**Abbildung 4** zeigt das System in Ausschnitten anhand von Fotoaufnahmen des in den Reaktor eingebrachten Siebs unterhalb des Rührers.

**Abbildung 5****:** Mischkennzahlen des Reaktorsystems mit und ohne eingebrachtes Sieb. Daten sind Mittelwerte aus jeweils drei Wiederholungen.

**Abbildung 6****:** Verweilzeitverteilungsfunktion des Systems mit integriertem Sieb. Daten sind jeweils Mittelwerte aus 3 Wiederholungen.

**Abbildung 7****:** Verweilzeitsummenfunktion des Systems mit integriertem Sieb. Daten sind jeweils Mittelwerte aus 3 Wiederholungen.

**Abbildung 8****:** Sauerstofftransferrate im System mit integriertem Sieb bei 100 upm. 0,5bar Druck im Modul. Daten sind jeweils Mittelwerte aus drei Wiederholungen.

**Abbildung 9****:** Mikroskopische Aufnahmen des Animpfprozesses im Abstand von 45 min nach Zugabe der Zellen.

**Abbildung 10****:** Mikroskopische Aufnahmen des Expansionsprozesses während der ersten drei Tage der Kultivierung.

**Abbildung 11****:** Schematische Darstellung des Prozessablaufs zur Ernte im System mit integriertem Sieb.

**Abbildung 12****:** Mikroskopische Aufnahmen der bewachsenen Carrier vor und nach der Ernte.

**Abbildung 13****:** Mikroskopische Aufnahmen der CellBeads von Zellen, die im System mit Sieb geerntet wurden.

## Patentansprüche

1. Vorrichtung zur Expansion und Ernte von Zellen oder zur Produktion von biopharmazeutischen Produkten durch adhärente Zelllinien, **dadurch gekennzeichnet, dass** die Vorrichtung in einen rührbaren Bioreaktor als Abtrennvorrichtung integriert werden kann.

2. Verfahren zur Expansion und Ernte von Zellen oder zur Produktion von biopharmazeutischen Produkten durch adhärente Zelllinien in einem rührbaren Bioreaktor mit einer integrierten Abtrennvorrichtung gemäß Anspruch 1.

3. Verfahren zur Expansion und Ernte von Zellen in einem Bioreaktor gemäß Anspruch 2 **gekennzeichnet durch** die Schritte:
- Bereitstellen eines Bioreaktors mit integrierter Abtrennvorrichtung gemäß Anspruch 1 mit geeignetem Medium und Carriern und Animpfen mit einer Starterkultur aus adhärent wachsenden Zellen;
- Messung der Sauerstoff-, Glukose- und/oder Laktatkonzentration, der Zelldichte, der Zellzahl, der Zellgrößenverteilung im Bioreaktor **durch** Impedanzspektroskopie sowie Monitoring des Animpfprozesses;
- Vergleich der Messwerte aus Schritt 2 mit Referenzwerten;
- Automatische Zufuhr von Nährstoffen im Perfusionsmodus oder Batch-Prozess **gekennzeichnet durch** scherarme blasenfreie Begasung **durch** Einsatz eines Membranmoduls oder **durch** Begasung mit reinem Sauerstoff;
- Ernte der Zellen **durch** Entleerung des Reaktors und Durchführung von Waschprozessen zur Entfernung von adhäsionsunterstützenden Komponenten im Medium **durch** Pufferlösung;
- Zugabe von Enzymlösung zur Zellablöse von den Carriern;
- nach Inkubationszeit Zugabe von Blocksubstanzen (z.B. Medium) zur Abstoppung des Ablöseprozesses und Blockierung des Enzyms;
- Auffangen der Zellsuspension in einem Erntegefäß;
- Konzentrierung der Zellsuspension.

4. Verfahren zur Produktion von biopharmazeutischen Produkten durch adhärente Zelllinien in einem Bioreaktor gemäß Anspruch 2 **gekennzeichnet durch** die Schritte:
- Bereitstellen eines Bioreaktors mit integrierter Abtrennvorrichtung mit geeignetem Medium und Carriern und Animpfen mit einer Starterkultur aus adhärent wachsenden Zellen;
- Messung der Sauerstoff-, Glukose- und/oder Laktatkonzentration, der Zelldichte, der Zellzahl, der Zellgrößenverteilung im Bioreaktor **durch** Impedanzspektroskopie sowie Monitoring des Animpfprozesses;
- Vergleich der Messwerte aus Schritt 2 mit Referenzwerten;
- Einsatz von Perfusionsmodi zur Reduktion von Produktinhibierung.
